# EUROPÄISCHE PATENTSCHRIFT

(11) **EP 1 180 023 B1**
(45) Veröffentlichungstag und Bekanntmachung des Hinweises auf die Patenterteilung: **01.12.2004**
(21) Anmeldenummer: 00929544.5
(22) Anmeldetag: 20.05.2000
(51) Int. Cl.: A61K 9/70

(54) **SUBSTRATABSCHNITTE MIT ERHÖHTER LAGERSTABILITÄT WÄHREND IHRER LAGERUNG IN BEUTELVERPACKUNG**
SUBSTRATE SECTIONS WITH IMPROVED STORAGE STABILITY DURING THEIR STORAGE IN BAGS
SECTIONS DE SUBSTRAT A STABILITE AU STOCKAGE AMELIOREE LORS DE LEUR STOCKAGE DANS DES SACS

(30) Priorität: 02.06.1999 DE 19925338
(43) Veröffentlichungstag der Anmeldung: 20.02.2002
(73) Patentinhaber: LTS Lohmann Therapie-Systeme AG, 56626 Andernach (DE)
(72) Erfinder: SCHUMANN, Klaus, D-56567 Neuwied (DE); THEOBALD, Frank, D-53498 Bad Breisig (DE); VON FALKENHAUSEN, Christian, D-53125 Bonn (DE)
(74) Vertreter: Flaccus, Rolf-Dieter, Dr.
(86) Internationale Anmeldenummer: PCT/EP2000/004588
(87) Internationale Veröffentlichungsnummer: WO 2000/074660

(56) Entgegenhaltungen:
- EP-A- 0 401 949
- EP-A- 0 413 487
- WO-A-92/10154
- WO-A-96/34633
- DE-A- 19 650 329

## Beschreibung

Die Erfindung betrifft gegen Wirkstoffverlust geschützt in Beuteln eingesiegelte, haftklebend ausgerüstete Substratabschnitte, deren Haftklebefläche durch eine sie mindestens zum Teil überragende Trägerschicht abgedeckt ist, die unter Ausbildung einer Ablösehilfe durch einen Einschnitt unterteilt ist, durch den während einer Lagerung Haftkleber im kalten Fluß austreten und eine Verklebung mit dem Beutel verursachen kann.

Die Herstellung und Verwendung flächig haftklebender Substratabschnitte ist bekannt. Bei diesen kann es sich z.B. um wirkstoffhaltige transdermale Therapiesysteme handeln. Wenn diese beispielsweise als Wirkstoffe flüchtige Bestandteile enthalten, ergibt sich die Notwendigkeit, die damit ausgebildeten Substratabschnitte im Zeitraum zwischen Herstellung und Anwendung, das heißt während ihrer Lagerung bis zur Applikation, vor dem verdampfen der flüchtigen Bestandteile zu schützen.
Dies wird beispielsweise dadurch erreicht, daß die haftklebenden Substratabschnitte zum Schutz ihrer haftklebenden Fläche mit einer ablösbaren Trägerschicht versehen und anschließend in einen sie allseitig umschließenden Beutel eingesiegelt werden. Zweckmäßigerweise überragt dabei die Trägerschicht die haftklebende Fläche eines Substratabschnittes, um dadurch einerseits das Abziehen der Trägerschicht vom Substratabschnitt vor Gebrauch zu erleichtern und andererseits ein Ankleben der Substratabschnitte an den Verpackungsflächen infolge in kaltem Fluß austretendem Haftklebematerial zu verhindern. Eine solche Verklebung würde die Entnahme des Laminats bei nur einseitig geöffneter Packung erschweren oder gar unmöglich machen.

Um hier Abhilfe zu schaffen, wurde bereits vorgeschlagen, in der ablösbaren Trägerschicht einen Einschnitt einzubringen, wodurch die Ablösung der Trägerschicht erleichtert und die Applikation der haftklebenden Substratabschnitte auf dem Applikationsort eines Patienten ermöglicht wird.
Ein Nachteil dieser Maßnahme ergibt sich daraus, daß während einer Lagerung der verpackten Substratabschnitte weiterhin durch kalten Fluß Haftkleber an der Schnittkante der ablösbaren Trägerschicht austreten und damit eine äußerst nachteilige Verklebung mit Verpackungsflächen verursachen kann, so daß die Entnahme des Laminats bei einseitig geöffneter Packung noch mehr erschwert bzw. unmöglich gemacht wird.

WO 96/ 34633 offenbart eine Wundauflage, die mit einer sie überlappenden und haftklebend beschichteten Rückschicht versehen ist, mit der das Pflaster auf der Haut des Patienten befestigt wird.

Der Erfindung liegt die Aufgabe zugrunde, bei einem Substratabschnitt der im Oberbegriff von Anspruch 1 genannten Art eine Ausbildung der mit einer Ablösehilfe versehenen Trägerschicht anzugeben, durch welche bei dieser ein Austreten von Haftkleber im kalten Fluß problemlos und mit unkomplizierten Mitteln und damit eine problemlose Entnahme von Substratabschnitten aus nur einseitig geöffneter Pakkung erleichtert wird.

Die Lösung der Aufgabe gelingt mit der Erfindung bei einem Substratabschnitt der eingangs genannten Art dadurch, daß zur Erhöhung der Lagerstabilität unter Vermeidung von Haftkleberaustritt die Trägerschicht mit zwei sich im Bereich ihrer Zusammensetzung überlappenden Trägerschichtabschnitten ausgebildet ist.

Mit großem Vorteil wird durch diese überraschend unkomplizierte Ausbildung der Trägerschicht ohne Einschränkung ihrer Ablösemöglichkeit vom Substratabschnitt ein Austritt von Haftkleber und damit Anhaften am Verpackungsmaterial vermieden und die Entnahme des Laminats bei nur einseitig geöffneter Packung problemlos erleichtert.

Ein verfahren zur Ausbildung von Substratabschnitten mit sie überragenden und einen Überlappungsbereich ausbildenden Trägerschichtabschnitten ist dadurch gekennzeichnet, daß die haftklebenden Substratabschnitte über ein Spendeverfahren, beispielsweise gemäß WO 92/17237, den Trägerschichtabschnitten nach deren Überlappung zugeführt werden.

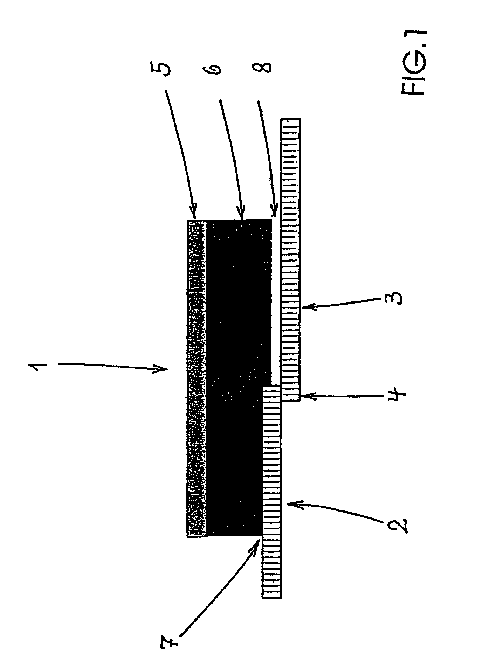

Weitere Einzelheiten, Merkmale und Vorteile der Erfindung ergeben sich aus der nachfolgenden Erläuterung eines in der Zeichnung schematisch dargestellten Ausführungsbeispieles. FIG.1 zeigt im Schnitt den Substratabschnitt 1, umfassend eine Rückschicht 5 und eine mit dieser verbundene Matrix 6. Diese kann entweder vollständig aus Haftklebematerial bestehen, oder an ihrer Applikationsfläche mit einer (nicht dargestellten) Haftklebeschicht haftklebend ausgerüstet sein. Die Haftklebefläche bzw. die haftklebend ausgebildete Matrix 6 ist durch eine sie mindestens zum Teil überragende Trägerschicht abgedeckt, die nach der Erfindung mit zwei sich im Bereich ihrer Zusammensetzung sie überlappenden Trägerschichtabschnitten 2,3 ausgebildet ist.
Dadurch, daß in an sich bekannter Weise die Trägerschichtabschnitte 2, 3 an den Seitenkanten 7, 8 der Matrix 6 diese überragen, wird bei möglichem Austritt von Haftkleber im kalten Fluß ein Anhaften an einem Verpackungsbeutel sicher verhindert.
Die aus Gründen der besseren Ablösbarkeit zweiteilig zusammengesetzte Trägerschicht 2, 3 verhindert infolge ihrer Ausbildung mit einem Überlappungsbereich 4 auch dort den Austritt von Haftkleber im kalten Fluß während einer längeren Lagerung.

Eine Ausgestaltung sieht vor, daß einer der Trägerschichtabschnitte 2 um den Betrag der Überlappung 4 breiter ist als der andere Abschnitt 3.

Es kann aber auch von der Maßnahme Gebrauch gemacht sein, daß beide Trägerschichtabschnitte 2, 3 um den Betrag der halben Überlappung breiter sind, als das halbe Breitenmaß einer ungeteilten Trägerschicht 1.

Weiterhin kann der Überlappungsbereich 4 ohne Schmälerung seiner Funktion als Ablösehilfe entweder mittig oder außermittig auf dem Substratabschnitt 1 abgeordnet sein. Diese Maßnahme erleichtert die Herstellung und deren Kontrolle.

Die Lösung gemäß vorliegender Erfindung ist überraschend einfach und löst in optimaler Weise die eingangs gestellte Aufgabe.

## Patentansprüche

1. Gegen Wirkstoffverlust geschützt in Beuteln eingesiegelte Substratabschnitte, die eine Rückschicht und eine mit dieser verbundene Matrix umfassen, welche vollständig aus einem Haftklebematerial besteht oder an ihrer Applikationsfläche mit einer Haftklebeschicht haftklebend ausgerüstet ist, wobei deren Haftklebefläche durch eine sie mindestens zum Teil überragende Trägerschicht abgedeckt ist, die unter Ausbildung einer Ablösehilfe durch einen Einschnitt unterteilt ist, durch den während einer Lagerung Haftkleber im kalten Fluß austreten und eine Verklebung mit dem Beutel verursachen kann, **dadurch gekennzeichnet, dass** zur Erhöhung der Lagerstabilität unter Vermeidung von Haftkleberaustritt die Trägerschicht mit zwei sich im Bereich ihrer Zusammensetzung überlappenden Trägerschichtabschnitten ausgebildet ist.

2. Substratabschnitt nach Anspruch 1, **dadurch gekennzeichnet, daß** einer der Trägerschichtabschnitte um den Betrag der Überlappung breiter ist als der andere Abschnitt.

3. Substratabschnitt nach Anspruch 1, **dadurch gekennzeichnet, daß** beide Trägerschichtabschnitte um den Betrag der halben Überlappung breiter sind als das halbe Breitenmaß einer ungeteilten Trägerschicht.

4. Substratabschnitt nach einem der Ansprüche 1 bis 3, **dadurch gekennzeichnet, daß** der Überlappungsbereich mittig oder außermittig auf dem Substratabschnitt angeordnet ist.

## Claims

1. Substrate sections which are sealed in bags so as to be protected against loss of active substance and comprising a backing layer and a matrix which is connected with said backing layer and is comprised entirely of a pressure-sensitive adhesive material or has been rendered pressure-sensitive adhesive on its application surface by providing said surface with a pressure-sensitive adhesive layer, the pressure-sensitive adhesive surface of said matrix being covered by a carrier layer which projects at least partially beyond said substrate sections and is divided by an incision, thus forming a detachment aid, through which incision adhesive may leak out in cold flow during storage and may cause the substrate section to stick to the bag, **characterized in that** in order to increase storage stability by avoiding leakage of pressure-sensitive adhesive, the carrier layer is configured with two carrier layer sections which overlap each other in the region where they are joined.

2. Substrate section according to Claim 1, **characterized in that** one of the carrier layer sections is wider than the other section by the amount of the overlap.

3. Substrate section according to Claim 1, **characterized in that** both carrier layer sections are, by the amount of half the overlap, wider than half the width dimension of an undivided carrier layer.

4. Substrate section according to Claims 1 to 3, **characterized in that** the region of overlap is positioned centrally or eccentrically on the substrate section.

## Revendications

1. Sections de substrat protégées contre les pertes de matières, scellées dans des sacs et qui comportent une couche arrière à laquelle est reliée une matrice entièrement constituée d'un matériau adhésif ou rendue adhésive sur sa surface d'application par une couche adhésive, la surface adhésive étant recouverte d'une couche de support qui en déborde au moins en partie et qui est divisée par une entaille qui forme un accessoire de séparation et par laquelle, lors du stockage, des adhésifs sortent par ressuage à froid et peuvent entraîner une adhérence au sac, **caractérisées en ce que**, pour augmenter la stabilité au stockage tout en évitant le ressuage des adhésifs, la couche de support est constituée de deux sections de couche de support qui se superposent dans la région de leur assemblage.

2. Section de substrat selon la revendication 1, **caractérisée en ce que** la largeur de l'une des sections de la couche de support dépasse la largeur de l'autre section d'une valeur égale à la superposition.

3. Section de substrat selon la revendication 1, **caractérisée en ce que** la largeur de chacune des deux sections de la couche de support dépasse de la moitié de la superposition la moitié de la largeur d'une couche de support non divisée.

4. Section de substrat selon l'une quelconque des revendications 1 à 3, **caractérisée en ce que** la zone de superposition est agencée de façon centrée ou décentrée sur la section de substrat.
